# EUROPEAN PATENT APPLICATION

(11) **EP 1 033 403 A1**
(43) Date of publication of application: **06.09.2000**
(21) Application number: 98955963.8
(22) Date of filing: 27.11.1998
(51) Int. Cl.: C12N 15/12, C12Q 1/68, C12P 21/02, G01N 33/53, A61K 38/16

(54) **GENE ORIGINATING IN HUMAN FETAL CHONDROCYTES**

(30) Priority: 27.11.1997 JP 34206097
(71) Applicant: CHUGAI SEIYAKU KABUSHIKI KAISHA, Tokyo, 115-8543 (JP)
(72) Inventor: KATO, Yukio, Hiroshima-shi Hiroshima 732-0062 (JP); KAWAMOTO, Takeshi, Yoshiki-gun Yamaguchi 754-1200 (JP); KOYANO, Yasuhiko, Hiroshima-shi Hiroshima 734-0002 (JP)
(74) Representative: HOFFMANN - EITLE
(86) International application number: JP9805348
(87) International publication number: WO9928458

(57) **Abstract**

The present invention provides a human chondrocyte-derived gene. That is, a protein encoded by the human chondrocyte-derived gene according to the invention has a structure characteristic of a regulatory factor for a cytoskeleton binding to protein on a cell membrane, and also an epistatic factor for regulating the functions of Rho. In detail, the protein has all of an ezrin-like domain, a DH domain, and a PH domain.

SEQUENCE LISTING

<110> CHUGAI SEIYAKU KABUSHIKI KAISHA

<120> cDNA and deduced amino acid sequence in human fetus chondrocytes

<130> CGS98-04PCT

<160> 22

<170> PatentIn Ver. 2.0

<210> 1
<211> 3442
<212> DNA
<213> Homo sapiense

<220>
<221> CDS
<222> (49)..(3183)

<400> 1
cgccgcagcc gccggcgctg tggagatatt ctctaagccg ctttcatc atg gga gaa 57
Met Gly Glu
1

ata gag cag agg ccg acc cca gga tca cga ctg ggg gcc ccg gaa aat 105
Ile Glu Gln Arg Pro Thr Pro Gly Ser Arg Leu Gly Ala Pro Glu Asn
    5                   10                  15

tcg ggg atc agt acc ttg gaa cgt gga cag aag ccg ccc cca aca cct 153
Ser Gly Ile Ser Thr Leu Glu Arg Gly Gln Lys Pro Pro Pro Thr Pro
20                  25                  30                  35

tca gga aaa ctc gtg tcc atc aaa atc cag atg ctg gat gac acc cag 201
Ser Gly Lys Leu Val Ser Ile Lys Ile Gln Met Leu Asp Asp Thr Gln
                40                  45                  50

gag gca ttt gaa gtt cca caa aga gct cct ggg aag gtg ctg ctg gat 249
Glu Ala Phe Glu Val Pro Gln Arg Ala Pro Gly Lys Val Leu Leu Asp
            55                  60                  65

gca gtt tgc aac cac ctc aac ctc gtg gaa ggt gac tat ttt ggc ctc 297

Ala Val Cys Asn His Leu Asn Leu Val Glu Gly Asp Tyr Phe Gly Leu
        70                  75                  80

gag ttt cct gat cac aaa aag atc acg gtg tgg ctg gat ctc cta aaa    345
Glu Phe Pro Asp His Lys Lys Ile Thr Val Trp Leu Asp Leu Leu Lys
    85                  90                  95

ccc att gtg aaa cag att aga agg cca aag cac gtt gtt gtt aag ttt    393
Pro Ile Val Lys Gln Ile Arg Arg Pro Lys His Val Val Val Lys Phe
100                 105                 110                 115

gtg gtg aaa ttc ttt ccg cct gac cac aca caa ctc caa gaa gaa ctc    441
Val Val Lys Phe Phe Pro Pro Asp His Thr Gln Leu Gln Glu Glu Leu
                120                 125                 130

aca agg tac ctg ttc gcg ctg cag gtg aag cag gac ttg gct caa ggc    489
Thr Arg Tyr Leu Phe Ala Leu Gln Val Lys Gln Asp Leu Ala Gln Gly
            135                 140                 145

agg ttg acg tgt aat gac acc agc gca gct ctc ttg att tca cac att    537
Arg Leu Thr Cys Asn Asp Thr Ser Ala Ala Leu Leu Ile Ser His Ile
        150                 155                 160

gtg caa tct gag att ggg gat ttt gat gaa gcc ttg gac aga gag cac    585
Val Gln Ser Glu Ile Gly Asp Phe Asp Glu Ala Leu Asp Arg Glu His
    165                 170                 175

tta gca aaa aat aaa tac ata cct cag caa gac gca cta gag gac aaa    633
Leu Ala Lys Asn Lys Tyr Ile Pro Gln Gln Asp Ala Leu Glu Asp Lys
180                 185                 190                 195

atc gtg gaa ttt cac cat aac cac att gga caa aca cca gca gaa tca    681
Ile Val Glu Phe His His Asn His Ile Gly Gln Thr Pro Ala Glu Ser
                200                 205                 210

gat ttc cag ctc cta gag att gcc cgt cgg cta gag atg tat gga atc    729
Asp Phe Gln Leu Leu Glu Ile Ala Arg Arg Leu Glu Met Tyr Gly Ile
            215                 220                 225

cgg ttg cac ccg gcc aag gac agg gaa ggc acg aag atc aat ctg gcc    777
Arg Leu His Pro Ala Lys Asp Arg Glu Gly Thr Lys Ile Asn Leu Ala
        230                 235                 240

gtt gcc aac acg gga att cta gtg ttt cag ggt ttc act aag atc aat    825
Val Ala Asn Thr Gly Ile Leu Val Phe Gln Gly Phe Thr Lys Ile Asn

245                 250                 255

gcc ttc aac tgg gcc aag gtg cgg aag ctg agc ttc aag agg aag cgc     873
Ala Phe Asn Trp Ala Lys Val Arg Lys Leu Ser Phe Lys Arg Lys Arg
260                 265                 270                 275

ttt ctc atc aag ctc cgg cca gat gcc aat agt gcg tac cag gat acc     921
Phe Leu Ile Lys Leu Arg Pro Asp Ala Asn Ser Ala Tyr Gln Asp Thr
                280                 285                 290

ttg gaa ttc ctg atg gcc agt cgg gat ttc tgc aag tcc ttc tgg aaa     969
Leu Glu Phe Leu Met Ala Ser Arg Asp Phe Cys Lys Ser Phe Trp Lys
            295                 300                 305

atc tgt gtt gaa cat cat gcc ttc ttt aga ctt ttt gaa gag ccc aaa     1017
Ile Cys Val Glu His His Ala Phe Phe Arg Leu Phe Glu Glu Pro Lys
        310                 315                 320

cca aag ccc aag ccc gtc ctc ttt agc cgg ggg tca tca ttt cgg ttc     1065
Pro Lys Pro Lys Pro Val Leu Phe Ser Arg Gly Ser Ser Phe Arg Phe
    325                 330                 335

agt ggt cgg act cag aag cag gtt ctc gac tat gtt aaa gaa gga gga     1113
Ser Gly Arg Thr Gln Lys Gln Val Leu Asp Tyr Val Lys Glu Gly Gly
340                 345                 350                 355

cat aag aag gtg cag ttt gaa agg aag cac agc aag att cat tct atc     1161
His Lys Lys Val Gln Phe Glu Arg Lys His Ser Lys Ile His Ser Ile
                360                 365                 370

cgg agc ctt gct tca cag cct aca gaa ctg aat tcg gaa gtg ctg gag     1209
Arg Ser Leu Ala Ser Gln Pro Thr Glu Leu Asn Ser Glu Val Leu Glu
            375                 380                 385

cag tct cag cag agc acc agc ctt aca ttt gga gaa ggt gcc gaa tct     1257
Gln Ser Gln Gln Ser Thr Ser Leu Thr Phe Gly Glu Gly Ala Glu Ser
        390                 395                 400

cca ggg ggc cag agc tgc cgg cga gga aag gaa ccg aag gtt tcc gcc     1305
Pro Gly Gly Gln Ser Cys Arg Arg Gly Lys Glu Pro Lys Val Ser Ala
    405                 410                 415

ggg gag ccg ggg tcg cac ccg agc cct gcg ccg agg aga agc ccc gcg     1353
Gly Glu Pro Gly Ser His Pro Ser Pro Ala Pro Arg Arg Ser Pro Ala
420                 425                 430                 435

ggt aac aag cag gcg gac gga gcc gcc tcg gcg ccc acg gag gaa gag    1401
Gly Asn Lys Gln Ala Asp Gly Ala Ala Ser Ala Pro Thr Glu Glu Glu
                440                 445                 450

gag gag gtc gtt aag gat agg acc cag cag agt aaa cct cag ccc ccg    1449
Glu Glu Val Val Lys Asp Arg Thr Gln Gln Ser Lys Pro Gln Pro Pro
            455                 460                 465

cag cca agc aca ggc tcc ctg act ggc agt cct cac ctt tcc gag ctg    1497
Gln Pro Ser Thr Gly Ser Leu Thr Gly Ser Pro His Leu Ser Glu Leu
        470                 475                 480

tct gtg aac tcg cag ggg gga gtg gcc cct gcc aac gtg acc ttg tct    1545
Ser Val Asn Ser Gln Gly Gly Val Ala Pro Ala Asn Val Thr Leu Ser
    485                 490                 495

ccc aac ctg agc ccc gac acc aag cag gcc tct ccc ttg atc agc ccg    1593
Pro Asn Leu Ser Pro Asp Thr Lys Gln Ala Ser Pro Leu Ile Ser Pro
500                 505                 510                 515

ctg ctg aat gac cag gcc tgc ccc cgg acg gac gat gag gat gag ggc    1641
Leu Leu Asn Asp Gln Ala Cys Pro Arg Thr Asp Asp Glu Asp Glu Gly
                520                 525                 530

cgg agg aag aga ttc cca act gat aaa gcg tac ttc ata gct aag gaa    1689
Arg Arg Lys Arg Phe Pro Thr Asp Lys Ala Tyr Phe Ile Ala Lys Glu
            535                 540                 545

gtg tct acc acc gag cga aca tat ctg aag gat ctc gaa gtt atc act    1737
Val Ser Thr Thr Glu Arg Thr Tyr Leu Lys Asp Leu Glu Val Ile Thr
        550                 555                 560

tcg tgg ttt cag agc aca gtg agc aaa gag gac gcc atg ccg gaa gca    1785
Ser Trp Phe Gln Ser Thr Val Ser Lys Glu Asp Ala Met Pro Glu Ala
    565                 570                 575

ctg aaa agt ctc ata ttc ccg aat ttt gaa cct ttg cac aaa ttt cat    1833
Leu Lys Ser Leu Ile Phe Pro Asn Phe Glu Pro Leu His Lys Phe His
580                 585                 590                 595

act aat ttt ctc aag gaa att gag caa cga ctt gcc ctg tgg gaa ggc    1881
Thr Asn Phe Leu Lys Glu Ile Glu Gln Arg Leu Ala Leu Trp Glu Gly
                600                 605                 610

cgc tca aat gcc caa atc aga gat tac caa aga atc ggc gat gtc atg 1929
Arg Ser Asn Ala Gln Ile Arg Asp Tyr Gln Arg Ile Gly Asp Val Met
            615                 620                 625

ctg aag aac att cag ggc atg aag cac ctg gcg gct cac ctg tgg aag 1977
Leu Lys Asn Ile Gln Gly Met Lys His Leu Ala Ala His Leu Trp Lys
        630                 635                 640

cac agc gag gcc ttg gag gcc ctg gag aat gga atc aag agc tcc cgg 2025
His Ser Glu Ala Leu Glu Ala Leu Glu Asn Gly Ile Lys Ser Ser Arg
    645                 650                 655

cgg ctg gag aac ttc tgc aga gac ttt gag ctg cag aag gtg tgt tac 2073
Arg Leu Glu Asn Phe Cys Arg Asp Phe Glu Leu Gln Lys Val Cys Tyr
660                 665                 670                 675

cta ccg ctc aac acc ttc ctc ctg cgg cca ctg cac cgg ctc atg cac 2121
Leu Pro Leu Asn Thr Phe Leu Leu Arg Pro Leu His Arg Leu Met His
                680                 685                 690

tac aag cag gtc ctg gag cgg ctg tgc aaa cac cac ccg ccg agc cac 2169
Tyr Lys Gln Val Leu Glu Arg Leu Cys Lys His His Pro Pro Ser His
            695                 700                 705

gcc gac ttc agg gac tgc cga gcc gct ttg gca gag atc acg gag atg 2217
Ala Asp Phe Arg Asp Cys Arg Ala Ala Leu Ala Glu Ile Thr Glu Met
        710                 715                 720

gtg gca cag ctc cac ggt acg atg atc aag atg gag aat ttc cag aag 2265
Val Ala Gln Leu His Gly Thr Met Ile Lys Met Glu Asn Phe Gln Lys
    725                 730                 735

ctg cac gaa ctc aag aaa gat ttg att ggc att gac aat ctt gtg gtt 2313
Leu His Glu Leu Lys Lys Asp Leu Ile Gly Ile Asp Asn Leu Val Val
740                 745                 750                 755

ccg gga agg gag ttc atc cgt ctg ggc agc ctc agc aag ctc tcg ggg 2361
Pro Gly Arg Glu Phe Ile Arg Leu Gly Ser Leu Ser Lys Leu Ser Gly
                760                 765                 770

aag ggg ctc cag cag cgc atg ttc ttc ctg ttc aac gac gtc ctg cta 2409
Lys Gly Leu Gln Gln Arg Met Phe Phe Leu Phe Asn Asp Val Leu Leu
            775                 780                 785

tac acg agc cgg ggg ctg acg gcc tcc aat cag ttt aaa gtc cac ggg 2457

Tyr Thr Ser Arg Gly Leu Thr Ala Ser Asn Gln Phe Lys Val His Gly
        790                 795                 800

cag ctc ccg ctc tat ggc atg acg att gag gag agc gaa gac gag tgg     2505
Gln Leu Pro Leu Tyr Gly Met Thr Ile Glu Glu Ser Glu Asp Glu Trp
    805                 810                 815

ggg gtg ccc cac tgc ctg acc ctc cgg ggc cag cgg cag tcc atc atc     2553
Gly Val Pro His Cys Leu Thr Leu Arg Gly Gln Arg Gln Ser Ile Ile
820                 825                 830                 835

gtg gcc gcc agt tct cgg tcc gag atg gag aag tgg gtt gag gac atc     2601
Val Ala Ala Ser Ser Arg Ser Glu Met Glu Lys Trp Val Glu Asp Ile
                840                 845                 850

cag atg gcc att gac ctg gcg gag aag agc agc agc ccc gcc cct gag     2649
Gln Met Ala Ile Asp Leu Ala Glu Lys Ser Ser Ser Pro Ala Pro Glu
            855                 860                 865

ttc ctg gcc agc agc ccc cct gac aac aag tcc cct gat gaa gcc acc     2697
Phe Leu Ala Ser Ser Pro Pro Asp Asn Lys Ser Pro Asp Glu Ala Thr
        870                 875                 880

gcg gct gac cag gag tca gag gat gac ctg agc gcc tcg cgc aca tcg     2745
Ala Ala Asp Gln Glu Ser Glu Asp Asp Leu Ser Ala Ser Arg Thr Ser
    885                 890                 895

ctg gag cgc cag gcc ccg cac cgc ggc aac aca atg gtg cac gtg tgc     2793
Leu Glu Arg Gln Ala Pro His Arg Gly Asn Thr Met Val His Val Cys
900                 905                 910                 915

tgg cac cgc aac acc agc gtc tcc atg gtg gac ttc agc atc gca gtg     2841
Trp His Arg Asn Thr Ser Val Ser Met Val Asp Phe Ser Ile Ala Val
                920                 925                 930

gag aat cag ttg tct gga aac ctg ctg agg aaa ttc aaa aac agc aac     2889
Glu Asn Gln Leu Ser Gly Asn Leu Leu Arg Lys Phe Lys Asn Ser Asn
            935                 940                 945

ggg tgg cag aag ctg tgg gtg gtg ttc aca aac ttc tgc ctg ttc ttc     2937
Gly Trp Gln Lys Leu Trp Val Val Phe Thr Asn Phe Cys Leu Phe Phe
        950                 955                 960

tac aaa tca cac cag gac aat cat ccc ctt gcc agc ctg cct ctg ctc     2985
Tyr Lys Ser His Gln Asp Asn His Pro Leu Ala Ser Leu Pro Leu Leu

965                 970                 975

ggc tac tcg ctc acc atc ccc tct gag tcc gag aac atc cag aaa gac    3033
Gly Tyr Ser Leu Thr Ile Pro Ser Glu Ser Glu Asn Ile Gln Lys Asp
980                 985                 990                 995

tac gtg ttc aag ctg cac ttc aag tcc cac gtc tac tac ttc agg gcg    3081
Tyr Val Phe Lys Leu His Phe Lys Ser His Val Tyr Tyr Phe Arg Ala
                1000                1005                1010

gaa agc gag tac acg ttc gaa agg tgg atg gaa gtg atc cgc agt gcc    3129
Glu Ser Glu Tyr Thr Phe Glu Arg Trp Met Glu Val Ile Arg Ser Ala
            1015                1020                1025

acc agc tct gcc tcg cga ccc cac gtg ttg agc cac aaa gag tct ctt    3177
Thr Ser Ser Ala Ser Arg Pro His Val Leu Ser His Lys Glu Ser Leu
        1030                1035                1040

gtg tat tgatggccgg acacactcgt ttccgcagtg gctgctttcc tggaagacgt    3233
Val Tyr
    1045

ttcctttctt ctgtattaat gaagcctggt aaaattaaca cctgtctgaa aatcaaaaac 3293

atggcttccc agcagctctc ctgtctccac agccgcgttt tttaaccccg acctctcagc 3353

gtttgaatga acagcgctcc cacctccagt cctggcatcc gctgggggcg ctgttcttta 3413

gctagtgcca gtattaaaac attgtcatt                                   3442

<210> 2
<211> 1045
<212> PRT
<213> Homo sapiens

<400> 2
Met Gly Glu Ile Glu Gln Arg Pro Thr Pro Gly Ser Arg Leu Gly Ala
1               5                   10                  15

Pro Glu Asn Ser Gly Ile Ser Thr Leu Glu Arg Gly Gln Lys Pro Pro
            20                  25                  30

Pro Thr Pro Ser Gly Lys Leu Val Ser Ile Lys Ile Gln Met Leu Asp
        35                  40                  45

Asp Thr Gln Glu Ala Phe Glu Val Pro Gln Arg Ala Pro Gly Lys Val
    50                  55                  60

Leu Leu Asp Ala Val Cys Asn His Leu Asn Leu Val Glu Gly Asp Tyr
65                  70                  75                  80

Phe Gly Leu Glu Phe Pro Asp His Lys Lys Ile Thr Val Trp Leu Asp
                85                  90                  95

Leu Leu Lys Pro Ile Val Lys Gln Ile Arg Arg Pro Lys His Val Val
            100                 105                 110

Val Lys Phe Val Val Lys Phe Phe Pro Pro Asp His Thr Gln Leu Gln
        115                 120                 125

Glu Glu Leu Thr Arg Tyr Leu Phe Ala Leu Gln Val Lys Gln Asp Leu
    130                 135                 140

Ala Gln Gly Arg Leu Thr Cys Asn Asp Thr Ser Ala Ala Leu Leu Ile
145                 150                 155                 160

Ser His Ile Val Gln Ser Glu Ile Gly Asp Phe Asp Glu Ala Leu Asp
                165                 170                 175

Arg Glu His Leu Ala Lys Asn Lys Tyr Ile Pro Gln Gln Asp Ala Leu
            180                 185                 190

Glu Asp Lys Ile Val Glu Phe His His Asn His Ile Gly Gln Thr Pro
        195                 200                 205

Ala Glu Ser Asp Phe Gln Leu Leu Glu Ile Ala Arg Arg Leu Glu Met
    210                 215                 220

Tyr Gly Ile Arg Leu His Pro Ala Lys Asp Arg Glu Gly Thr Lys Ile
225                 230                 235                 240

Asn Leu Ala Val Ala Asn Thr Gly Ile Leu Val Phe Gln Gly Phe Thr
                245                 250                 255

Lys Ile Asn Ala Phe Asn Trp Ala Lys Val Arg Lys Leu Ser Phe Lys
            260                 265                 270

Arg Lys Arg Phe Leu Ile Lys Leu Arg Pro Asp Ala Asn Ser Ala Tyr
        275                 280                 285

Gln Asp Thr Leu Glu Phe Leu Met Ala Ser Arg Asp Phe Cys Lys Ser
    290                 295                 300

Phe Trp Lys Ile Cys Val Glu His His Ala Phe Phe Arg Leu Phe Glu
305                 310                 315                 320

Glu Pro Lys Pro Lys Pro Lys Pro Val Leu Phe Ser Arg Gly Ser Ser
                325                 330                 335

Phe Arg Phe Ser Gly Arg Thr Gln Lys Gln Val Leu Asp Tyr Val Lys
            340                 345                 350

Glu Gly Gly His Lys Lys Val Gln Phe Glu Arg Lys His Ser Lys Ile
        355                 360                 365

His Ser Ile Arg Ser Leu Ala Ser Gln Pro Thr Glu Leu Asn Ser Glu
    370                 375                 380

Val Leu Glu Gln Ser Gln Gln Ser Thr Ser Leu Thr Phe Gly Glu Gly
385                 390                 395                 400

Ala Glu Ser Pro Gly Gly Gln Ser Cys Arg Arg Gly Lys Glu Pro Lys
                405                 410                 415

Val Ser Ala Gly Glu Pro Gly Ser His Pro Ser Pro Ala Pro Arg Arg
            420                 425                 430

Ser Pro Ala Gly Asn Lys Gln Ala Asp Gly Ala Ala Ser Ala Pro Thr
        435                 440                 445

Glu Glu Glu Glu Glu Val Val Lys Asp Arg Thr Gln Gln Ser Lys Pro
    450                 455                 460

Gln Pro Pro Gln Pro Ser Thr Gly Ser Leu Thr Gly Ser Pro His Leu
465                 470                 475                 480

Ser Glu Leu Ser Val Asn Ser Gln Gly Gly Val Ala Pro Ala Asn Val
                485                 490                 495

Thr Leu Ser Pro Asn Leu Ser Pro Asp Thr Lys Gln Ala Ser Pro Leu
            500                 505                 510

Ile Ser Pro Leu Leu Asn Asp Gln Ala Cys Pro Arg Thr Asp Asp Glu
        515                 520                 525

Asp Glu Gly Arg Arg Lys Arg Phe Pro Thr Asp Lys Ala Tyr Phe Ile
    530                 535                 540

Ala Lys Glu Val Ser Thr Thr Glu Arg Thr Tyr Leu Lys Asp Leu Glu
545                 550                 555                 560

Val Ile Thr Ser Trp Phe Gln Ser Thr Val Ser Lys Glu Asp Ala Met
                565                 570                 575

Pro Glu Ala Leu Lys Ser Leu Ile Phe Pro Asn Phe Glu Pro Leu His
            580                 585                 590

Lys Phe His Thr Asn Phe Leu Lys Glu Ile Glu Gln Arg Leu Ala Leu
        595                 600                 605

Trp Glu Gly Arg Ser Asn Ala Gln Ile Arg Asp Tyr Gln Arg Ile Gly
    610                 615                 620

Asp Val Met Leu Lys Asn Ile Gln Gly Met Lys His Leu Ala Ala His
625                 630                 635                 640

Leu Trp Lys His Ser Glu Ala Leu Glu Ala Leu Glu Asn Gly Ile Lys
                645                 650                 655

Ser Ser Arg Arg Leu Glu Asn Phe Cys Arg Asp Phe Glu Leu Gln Lys
            660                 665                 670

Val Cys Tyr Leu Pro Leu Asn Thr Phe Leu Leu Arg Pro Leu His Arg
        675                 680                 685

Leu Met His Tyr Lys Gln Val Leu Glu Arg Leu Cys Lys His His Pro
    690                 695                 700

Pro Ser His Ala Asp Phe Arg Asp Cys Arg Ala Ala Leu Ala Glu Ile
705                 710                 715                 720

Thr Glu Met Val Ala Gln Leu His Gly Thr Met Ile Lys Met Glu Asn
                725                 730                 735

Phe Gln Lys Leu His Glu Leu Lys Lys Asp Leu Ile Gly Ile Asp Asn
            740                 745                 750

Leu Val Val Pro Gly Arg Glu Phe Ile Arg Leu Gly Ser Leu Ser Lys
        755                 760                 765

Leu Ser Gly Lys Gly Leu Gln Gln Arg Met Phe Phe Leu Phe Asn Asp
    770                 775                 780

Val Leu Leu Tyr Thr Ser Arg Gly Leu Thr Ala Ser Asn Gln Phe Lys
785                 790                 795                 800

Val His Gly Gln Leu Pro Leu Tyr Gly Met Thr Ile Glu Glu Ser Glu
                805                 810                 815

Asp Glu Trp Gly Val Pro His Cys Leu Thr Leu Arg Gly Gln Arg Gln
            820                 825                 830

Ser Ile Ile Val Ala Ala Ser Ser Arg Ser Glu Met Glu Lys Trp Val
        835                 840                 845

Glu Asp Ile Gln Met Ala Ile Asp Leu Ala Glu Lys Ser Ser Ser Pro
    850                 855                 860

Ala Pro Glu Phe Leu Ala Ser Ser Pro Pro Asp Asn Lys Ser Pro Asp
865                 870                 875                 880

Glu Ala Thr Ala Ala Asp Gln Glu Ser Glu Asp Asp Leu Ser Ala Ser
                885                 890                 895

Arg Thr Ser Leu Glu Arg Gln Ala Pro His Arg Gly Asn Thr Met Val
            900                 905                 910

His Val Cys Trp His Arg Asn Thr Ser Val Ser Met Val Asp Phe Ser
        915                 920                 925

Ile Ala Val Glu Asn Gln Leu Ser Gly Asn Leu Leu Arg Lys Phe Lys
    930                 935                 940

Asn Ser Asn Gly Trp Gln Lys Leu Trp Val Val Phe Thr Asn Phe Cys
945                 950                 955                 960

Leu Phe Phe Tyr Lys Ser His Gln Asp Asn His Pro Leu Ala Ser Leu
                965                 970                 975

Pro Leu Leu Gly Tyr Ser Leu Thr Ile Pro Ser Glu Ser Glu Asn Ile
            980                 985                 990

Gln Lys Asp Tyr Val Phe Lys Leu His Phe Lys Ser His Val Tyr Tyr
        995                 1000                1005

Phe Arg Ala Glu Ser Glu Tyr Thr Phe Glu Arg Trp Met Glu Val Ile
   1010                    1015                    1020

Arg Ser Ala Thr Ser Ser Ala Ser Arg Pro His Val Leu Ser His Lys
025                    1030                    1035                    1040

Glu Ser Leu Val Tyr
                1045

<210> 3
<211> 25
<212> DNA
<213> Artificial

<220>
<223> probe

<400> 3
cataccggta agtggggcaa gactg 25

<210> 4
<211> 20
<212> DNA
<213> Artificial

<220>
<223> probe

<400> 4
tgcccagttc aggtctctta 20

<210> 5
<211> 20
<212> DNA
<213> Artificial

<220>
<223> probe

<400> 5
tgctacttca tcgaccccat 20

<210> 6
<211> 20

<212> DNA
<213> Artificial

<220>
<223> probe

<400> 6
aaagacctca ccctccatct 20

<210> 7
<211> 20
<212> DNA
<213> Artificial

<220>
<223> probe

<400> 7
gtcgattacg tggagagcta 20

<210> 8
<211> 20
<212> DNA
<213> Artificial

<220>
<223> probe

<400> 8
atgaacttct tcaccagctc 20

<210> 9
<211> 20
<212> DNA
<213> Artificial

<220>
<223> probe

<400> 9
gtcaaggctg agaacgggaa 20

<210> 10

<211> 20

<212> DNA
<213> Artificial

<220>
<223> probe

<400> 10
tccaccaccc tgttgctgta 20

<210> 11

<211> 20
<212> DNA
<213> Artificial

<220>
<223> probe

<400> 11
atcagaccca gctcccaaag 20

<210> 12

<211> 20
<212> DNA
<213> Artificial

<220>
<223> probe

<400> 12
cacagaccca gctcccaaac 20

<210> 13

<211> 20
<212> DNA
<213> Artificial

<220>
<223> probe

<400> 13
ccttcaggaa aactcgtgtc 20

<210> 14

<211> 20
<212> DNA
<213> Artificial

<220>
<223> probe

<400> 14
ttggagttgt gtgtggtcag 20

<210> 15

<211> 25
<212> DNA
<213> Artificial

<220>
<223> probe

<400> 15
gccaaaatag tcaccttcca cgagg 25

<210> 16

<211> 20
<212> DNA
<213> Artificial

<220>
<223> probe

<400> 16
ccttcaggaa aactcgtgtc 20

<210> 17

<211> 29
<212> DNA
<213> Artificial

<220>
<223> probe

<400> 17
aaacgraaga aygtrtgrtg ytcwacaca 29

<210> 18

<211> 20
<212> DNA
<213> Artificial

<220>
<223> probe

<400> 18
ttccagctcc tagagattgc 20

<210> 19

<211> 20
<212> DNA
<213> Artificial

<220>
<223> probe

<400> 19
tcgtcttcgc tctcctcaat 20

<210> 20

<211> 23
<212> DNA
<213> Artificial

<220>
<223> probe

<400> 20
cgggtaacaa gcaggcggac gga 23

<210> 21

<211> 20
<212> DNA
<213> Artificial

<220>
<223> probe

<400> 21
tcacttcgtg gtttcagagc 20

<210> 22

<211> 20
<212> DNA
<213> Artificial

<220>
<223> probe

<400> 22
tcgtcttcgc tctcctcaat 20

## Description

### Technical Field

This invention relates to a gene expressed specifically in differentiated human chondrocytes, a protein encoded by the gene, and an antibody against the protein.

### Background Art

No methods for monolayer culturing human chondrocytes in a differentiated state have been established. This has made it difficult to search for a gene specifically expressed in differentiated chondrocytes, and analyze the properties of a protein encoded by the gene.

### Disclosure of the Invention

Recently, the inventor of the present invention has discovered a method of monolayer culture for human chondrocytes in a differentiated state (M. Shen, T. Kawamoto, W. Yan, K. Nakamasu, M. Tamagami, Y. Koyano, M. Noshiro, and Y. Kato, Biochemical and Biophysical Research Communications 236, 294-298 (1997)). The inventor has found that a gene specifically expressed in human chondrocytes in a differentiated state can be searched for by using such a culture method.

Based on these findings, the inventor looked for genes different in expression between human chondrocytes in a differentiated state and chondrocytes in a dedifferentiated state, and found out a gene specifically expressed in former cells. This finding led to the present invention.

That is, the invention provides a gene expressed specifically in differentiated cells (cells in various animals, especially cells including chondrocytes and human chondrocytes), a protein encoded by the gene, and an antibody against the protein. The invention also provides the gene or its fragment, nucleotide sequences complementary to them, the protein or its mutant product, fragments thereof, a kit and a method for detecting differentiated chondrocytes with the use of the antibody, or a kit and a method for screening a regulator of cell differentiation induction.

More particularly, the invention provides [1] a protein specifically expressed in differentiated chondrocytes, including an ezrin-like domain, a Db1 domain, and a pleckstrin domain. The invention also provides [2] a protein having an amino acid sequence set forth in SEQ ID NO: 2 in the sequence listing. The invention also provides [3] a protein comprising the amino acid sequence set forth in SEQ ID NO: 2 in the sequence listing in which one to several amino acids have been deleted, substituted or added, the protein being specifically expressed in differentiated chondrocytes, and the protein being such that (1) an amino acid sequence of a portion of the protein corresponding to an amino acid sequence ranging from the 1st to 374th amino acids in SEQ ID NO: 2 in the sequence listing has homology of 85% or more to the amino acid sequence ranging from the 1st to 374th amino acids in the SEQ ID NO: 2, (2) an amino acid sequence of a portion of the protein corresponding to an amino acid sequence ranging from the 544th to 737th amino acids in SEQ ID NO: 2 in the sequence listing has homology of 85% or more to the amino acid sequence ranging from the 544th to 737th amino acids in the SEQ ID NO: 2, and (3) an amino acid sequence of a portion of the protein corresponding to an amino acid sequence ranging from the 764th to 854th amino acids in SEQ ID NO: 2 in the sequence listing has homology of 85% or more to the amino acid sequence ranging from the 764th to 854th amino acids in the SEQ ID NO: 2.

Further, the invention provides [4] DNA encoding the protein described in any one of the [1] to [3]. The invention also provides [5] a gene comprising DNA shown in the following (a) or (b): (a) DNA comprising a nucleotide sequence ranging from the 49th to 3,183rd bases in a nucleotide sequence set forth in SEQ ID NO: 1 in the sequence listing. (b) DNA which is hybridized under stringent conditions with DNA having a nucleotide sequence ranging from the 49th to 3,183rd bases in a nucleotide sequence set forth in SEQ ID NO: 1 in the sequence listing, and which encodes a protein specifically expressed in differentiated chondrocytes. The invention also provides [6] DNA having a part of or all of a nucleotide sequence set forth in SEQ ID NO: 1 in the sequence listing, or having a nucleotide sequence complementary to them.

Further, the invention provides [7] an antibody against the protein described in any one of [1] to [3].

Further, the invention provides [8] a kit for detecting differentiated chondrocytes, characterized by containing at least one of the following (a) to (c) as an active ingredient: (a) The protein described in any one of [1] to [3]. (b) The DNA described in any one of [4] to [6]. (c) The antibody described in [7]. The invention also provides [9] a method for detecting differentiated chondrocytes, characterized by using the kit described in [8].

Further, the invention provides [10] a kit for screening a regulator of cell differentiation induction, characterized by containing at least one of the following (a) to (c) as an active ingredient: (a) The protein described in any one of [1] to [3]. (b) The DNA described in any one of [4] to [6]. (c) The antibody described in [7]. The invention also provides [11] a method for screening a regulator of cell differentiation induction, characterized by using the kit described in [10].

Further, the invention provides [12] a screening method in which the regulator of cell differentiation induction described in [11] is an anti-tumor agent.

The amino acid sequence of the protein according to the present invention contains three domains, i.e., ezrin-like domain, Dbl homology (DH) domain, and pleckstrin homology (PH) domain, arranged in this order from the N-terminus in one molecule. Hence, this novel protein may be a very important factor for controlling the activity of an Rho family which exists near the cell membrane and regulates the function of the actin-based cytoskeleton responsible for the maintenance of cell shape and cell motility.

It becomes possible, moreover, to develop a drug for regulating cell differentiation induction or the proliferation of cancer, by activating or inhibiting the function of the protein according to the invention.

The invention also makes it possible to develop a probe indispensable for the analysis of the mechanism of cartilage differentiation or degeneration. The invention further permits the development of gene therapies of osteoarthritis and rheumatoid arthritis.

### Brief Description of the Drawings

Fig. 1 is a view showing a nucleic acid nucleotide sequence of CDEP cDNA, and an amino acid sequence deduced therefrom, in which ezrin-like domain is indicated by an open frame, Dbl homology (DH) domain is underlined, pleckstrin homology (PH) domain is double-underlined, an asterisk downstream from a protein coding region represents a stop codon, a poly(A) addition signal is indicated by a dashed line, and a poly(A) addition site is indicated by a triangle;
Fig. 2A is an electrophoretogram showing the results of northern blotting analysis of CDEP mRNA in various fetal human tissues, in which Ch- denotes a Bt2cAMP-untreated fibroblastic chondrocytes, Ch+ denotes a Bt2cAMP-dependent differentiated chondrocytes; Br denotes brain; He denotes heart; Li denotes liver; Sp denotes spleen; In denotes intestine; Lu denotes lung; Te denotes testis; Pl denotes placenta; Mu denotes muscle, and the size of CDEP mRNA is expressed in kb;
Fig. 2B is an electrophoretogram showing the results of northern blotting analysis of CDEP mRNA in various adult tissues, in which Ch- denotes a Bt2cAMP-untreated fibroblastic chondrocytes, Ch+ denotes a Bt2cAMP-dependent differentiated chondrocytes; Br denotes brain; He denotes heart; Li denotes liver; Sp denotes spleen; In denotes intestine; Lu denotes lung; Te denotes testis; Pl denotes placenta; Mu denotes muscle, and the size of CDEP mRNA is expressed in kb;
Fig. 3 is a view showing the arrangement of an ezrin-like domain and a Db1 homology (DH) domain, and comparisons among ezrin-like domain of human CDEP, ezrin, and band 4.1, in which the residues conserved with respect to CDEP are indicated in bold type;
Fig. 4 is a view showing comparisons among DH domain of human CDEP, human Dbl, rat Ost, mouse Est2, and human FGD1, in which the residues conserved with respect to CDEP are indicated in bold type;
Fig. 5 is a schematic view showing the relation between CDEP and other proteins;
Fig. 6 is an electrophoretogram showing the results of RT-PCR (reverse transcription PCR) of mRNA of type II collagen, aggrecan, cartilage matrix protein (CMP), glyceraldehyde-3-phosphate dehydrogenase (GAPDH) (upper row), DEC1 and CDEP (lower row), expressed in response to Bt₂cAMP in fetal human chondrocytes, in which cAMP+ represents the presence of Bt2cAMP, while cAMP- represents the absence of Bt2cAMP;
Fig. 7 shows the expression of CDEP, type X collagen, ALP, and aggrecan in each of R, G1, G2 and G3 in Example 4;
Fig. 8 shows the expression of CDEP in each stage of differentiated chondrocytes, and the effect of PTH addition on the expression;
Fig. 9 shows the time courses of CDEP expression after addition of Bt2cAMP and PTH;
Fig. 10 shows the time course of CDEP expression after addition of PTH;
Fig. 11A shows the results of immunofluorescence staining (under CNF2(-) conditions) indicating the localization of CDEP in the cell; and
Fig. 11B shows the results of immunofluorescence staining (under CNF2(+) conditions) indicating the localization of CDEP in the cell.

### Best Mode for Carrying Out the Invention

### (Protein, DNA encoding the protein)

The protein provided by the present invention is derived from DNA discovered by the invention that is set forth in SEQ ID NO: 1 in the sequence listing. A preferred example of the protein has an amino acid sequence set forth in SEQ ID NO: 2 in the sequence listing. As will be described later on in Examples, this sequence was obtained for the first time as a result of search for a gene expressed specifically in human chondrocytes in a differentiated state. The protein having this derived amino acid sequence is called CDEP in the present specification. As will be described below, proteins having different amino acids from those of the above protein, but having substantially the same function as this protein are also collectively called CDEP.

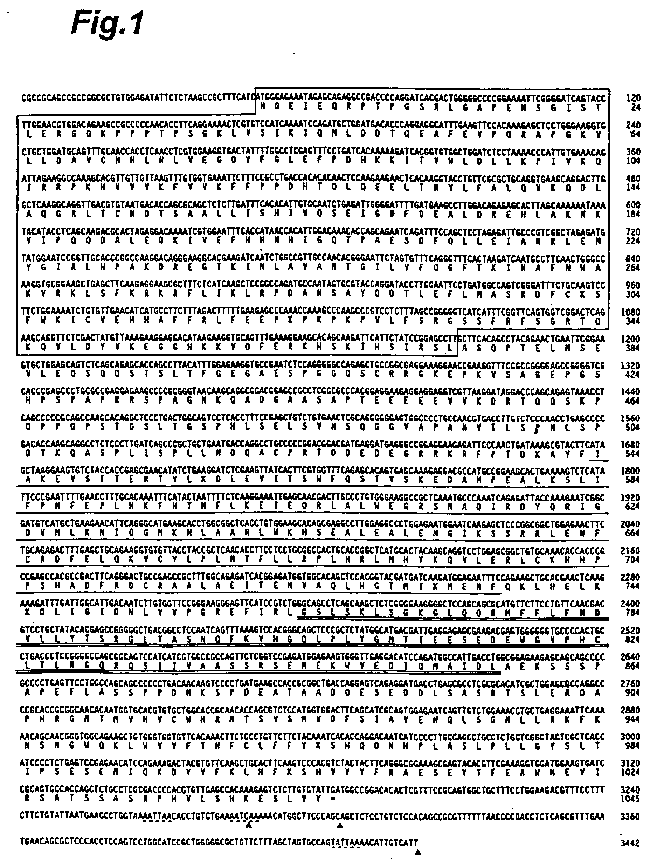

The total nucleotide sequence obtained as the gene encoding the protein CDEP (the gene will be referred to as CDEP cDNA) is described in SEQ ID NO: 1 in the sequence listing. The correspondences with amino acids deduced from the resulting nucleotide sequence are set forth in SEQ ID NO: 1 in the sequence listing, and Fig. 1 (amino acids are showed as three characters).

CDEP cDNA includes an open reading frame of 3135 bp starting with the first ATG codon existent on the 49th nucleotide. Since an in-frame stop codon is present upstream from the 5'-region (the 34th nucleotide), moreover, the first ATG is defined as an initiation codon. Thus, CDEP is assumed to comprise 1,045 amino acid residues, and its molecular weight is calculated at 118.6 kDa.

The amino acid sequence of CDEP deduced from the above nucleotide sequence is described in SEQ ID NO: 2 in the sequence listing, and Fig. 1. Based on the resulting amino acid sequence, the amino acid sequence of CDEP is found to lack a signal peptide for secretion, and a hydrophobic region of a transmembrane or membrane-bound domain.

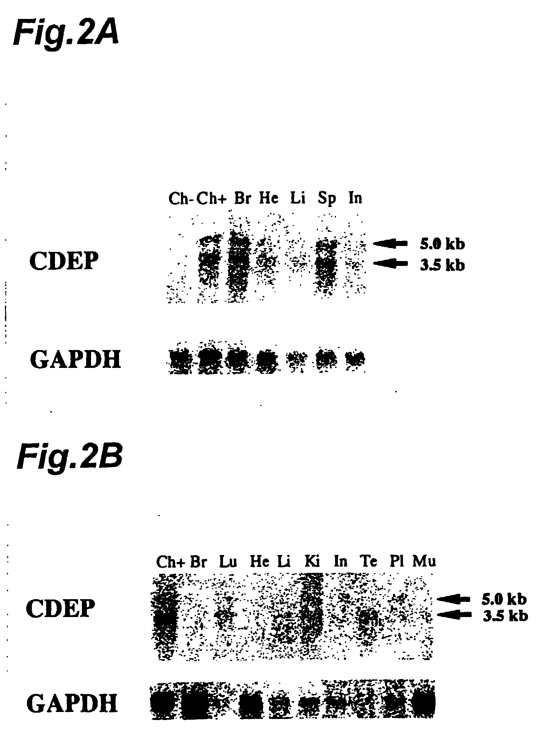

At least three polyadenylation signals and polyadenylation sites are found to exist. The total length of 3287 bp, 3306 bp or 3442 bp, excluding the poly(A) tract, is consistent with the size of mRNA (3.5 kb, indicated by an arrow as 3.5kb in Fig. 2(a)) determined by northern blotting analysis to be described below.

Homology search of the above amino acid sequence by the use of protein database has shown that the CDEP protein includes all of three functional domains, i.e., ezrin-like domain, DH domain, and PH domain (Figs. 1, 3, 4). No protein has been known which possesses all of ezrin-like domain, DH domain, and PH domain.

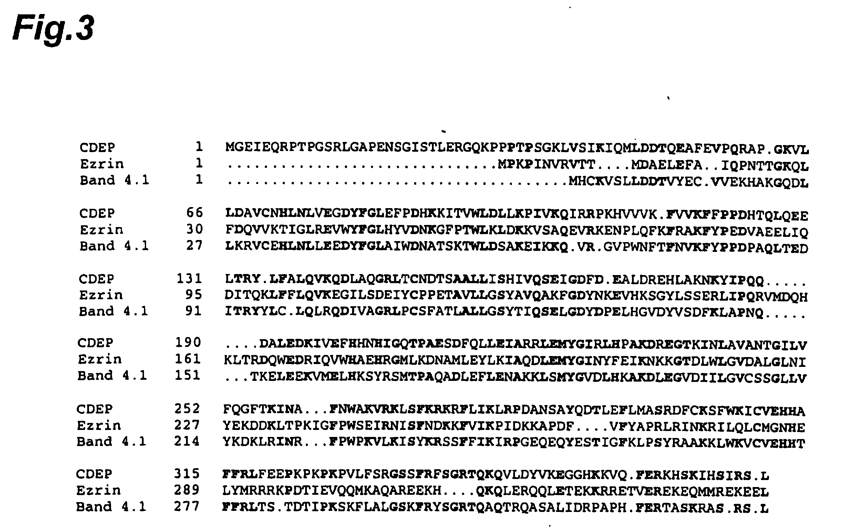

The ezrin-like domain that CDEP has refers to the one conserved in a band 4.1 superfamily, including ezrin, radixin, moesin, and band 4.1 that have been known. Fig. 3 shows the homology of the ezrin-like domain of CDEP to them. The ezrin-like domain of CDEP has homology of 27.5% to ezrin, and homology of 43.7% to band 4.1.

A protein such as ezrin is believed to play the role of a linker protein between the cytoskeleton and the cell membrane. Of some proteins recently reported, including an ezrin-like domain in the N-terminal region, merlin is known to be an tumor suppressor molecule which causes neurofibroma type 2, and PTPMEG1 and PTPH1 are known to be members of a cytoplasmic protein tyrosine phosphatase (PTP) family. These proteins are known to take part in the transmission of signals from outside of the cell to the inside of the cell at the boundary between the cell membrane and the cytoskeleton. A notable fact is that ezrin, radixin, and moesin (these are called ERM proteins) bind to a cytoplasmic domain of CD44, a hyaluronate receptor, through their N-terminal region. Also noticeable is that the binding of the ERM proteins to CD44 is enhanced by GTPγS, and markedly suppressed by C3 toxin, a specific inhibitor of Rho. As noted from these facts, GTP-Rho is required for the formation of a CD44/ERM protein complex.

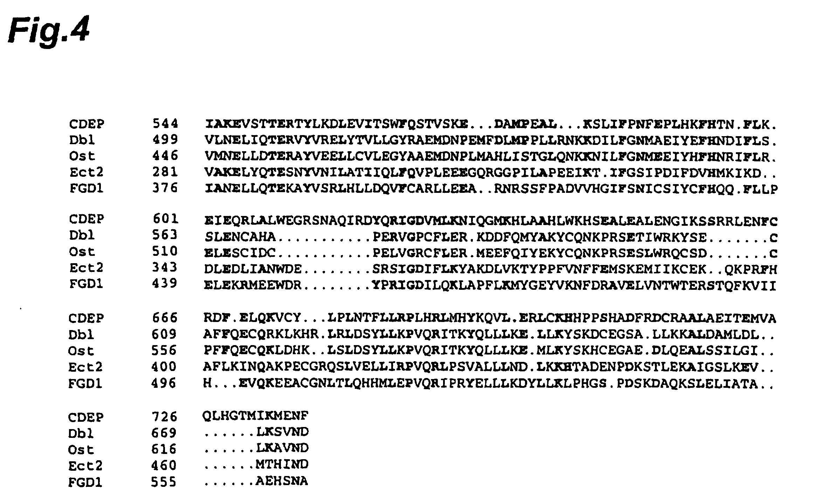

The CDEP protein of the invention also has the DH domain and the PH domain. The Rho GEF family, including Db1, Ost, Ect2 and Lbc which are oncogene products, and FGD1 which is a causative gene for faciogenital dysplasia, is also known to include a DH domain, and to be bound directly to a PH domain (DH-PH sequence) at the C-terminus. The homology of CDEP in terms of DH domain is 22.9% for Db1, 22.9% for Ost, 22.3% for Ect2, and 25.6% for FGD1 (Fig. 4).

Rho GEF is known to convert GDP-Rho (inactive type), in particular, into GTP-Rho (active type), while Ras GEFs are known to convert GDP-Ras into GTP-Ras. Rho and Ras belong to a small GTP-binding protein superfamily, and are known to take part in intracellular signal transduction. The superfamily is divided into five subfamilies, i.e., Ras, Rho, Rab, Arf, and other. The Rho subfamily (Rho, Rac, and Cdc42) is known to control the morphology, migration, aggregation and adhesion of cells by contraction of actin filaments. It is also known that some regulators of Rho, such as GEFs and GTPase-activating proteins (GAPs), are present upstream in the Rho cascade.

It is further known that the DH-PH domain is necessary for Rho GEF activity, while the Cdc-25 domain is necessary for Ras GEF activity; some of Ras GEF family members, such as Ras-GRF and Sos, have Ras GEF activity, but have no Rho GEF activity, even though they have DH-PH domain in addition to Cdc-25 domain.

As described above, CDEP of the invention is characterized by lacking the Cdc-25 domain and containing the DH-PH domain. Thus, CDEP is one of the members of the Rho GEF family, rather than the Ras GEP family.

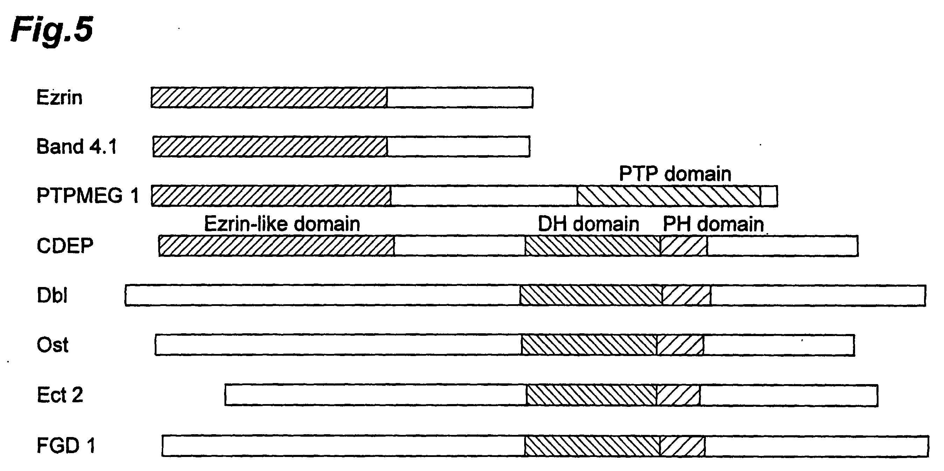

The ERM proteins having the ezrin-like domain are known to be involved in signal transduction leading to morphological changes of cells through the formation of a CD44/ERM/actin complex. Formation of this complex requires the activity of Rho by Rho GEF. CDEP obtained in the invention is found to be a very unique protein containing an ezrin-like domain and an Rho GEF-like domain in one molecule (Fig. 5). This is suggestive of a possibility that this substance will modify signal transduction in the CD44 path and/or a path mediated by other receptor. Furthermore, many members of the Rho GEF family become oncogenes for lack of the N-terminal region. This finding suggests that CDEP plays an important role in controlling the adhesion, diffusion, migration, proliferation, and differentiation of cells, including chondrocytes.

Therefore, the CDEP protein of the invention includes not only a protein having the amino acid sequence set forth in SEQ ID NO: 2, but also a protein having a novel amino acid sequence comprising the amino acid sequence of SEQ ID NO. 2 in which one to several amino acids have been deleted, substituted or added, the novel amino acid sequence having an ezrin-like domain, a DH domain, and a PH domain in the molecule.

Particularly, the CDEP protein includes a protein in which an amino acid sequence ranging from the 1st to 373rd amino acids in SEQ ID NO: 2 (called ezrin-like domain) has homology of 70% or more (preferably 85% or more) to the amino acid sequence of the SEQ ID NO: 2, in which an amino acid sequence ranging from the 544th to 737th amino acids in SEQ ID NO: 2 (called DH domain) has homology of 70% or more (preferably 85% or more) to the amino acid sequence of the SEQ ID NO: 2, and in which an amino acid sequence ranging from the 764th to 854th amino acids in SEQ ID NO: 2 (called PH domain) has homology of 70% or more (preferably 85% or more) to the amino acid sequence of the SEQ ID NO: 2.

The protein having such amino acid sequences has substantially the same domains as ezrin-like, DH and PH domains characteristic of CDEP, and is thus presumed to have substantially the same biochemical functions as does CDEP.

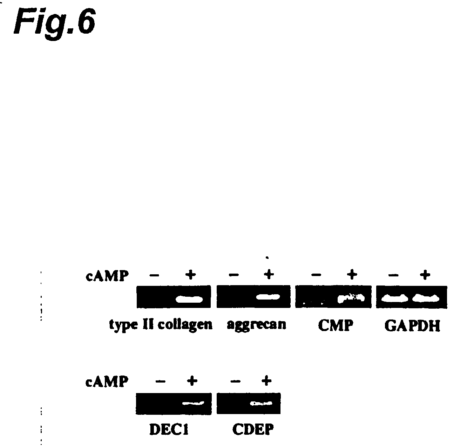

The CDEP protein, which has a different amino acid sequence from that of CDEP having the amino acid sequence described in SEQ ID NO: 2 in the sequence listing and which has substantially the same functions, can be obtained by a method for replacing, deleting, or inserting particular amino acid residues in the amino acid sequence described in SEQ ID NO: 2 in the sequence listing. Such a method is not restricted, and a publicly known method can be used preferably as this method. For example, the CDEP protein can be formed by introducing a mutation, such as substitution, deletion or insertion of nucleotide, into the nucleotide sequence by a publicly known method such as site-directed mutagenesis, and people skilled in the art can easily select the type of the mutant protein. That is, derivatives having functions comparable to those of particular proteins can be prepared by any methods of chemical peptide synthesis well known in the art, or ordinary genetic engineering methods, for instance, recombinant DNA technologies based on DNA sequence as disclosed in Sambrook et al., Molecular Cloning, Cold Spring Harbour Laboratory Press, New York, USA, Second Edition, 1989. Concretely, the method described, for example, in H. Neurath R.L. Hill, "The Proteins" (Academic Press, New York, 1979, especially in Fig. 6 on page 14) is usable. Substitutions that occur most usually are Ala/Ser, Val/Ile, Asp/Glu, Thr/Ser, Ala/Gly, Ala/Thr, Ser/Asn, Ala/Val, Ser/Gly, Tyr/Phe, Ala/Pro, Lys/Arg, Asp/Asn, Leu/Ile, Leu/Val, Ala/Glu and Asp/Gly, and vice versa.

Thus, DNA encoding the CDEP protein includes not only that coding for a protein having the amino acid sequence set forth in SEQ ID NO: 2, but also DNA encoding a protein having a novel amino acid sequence comprising the amino acid sequence of SEQ ID NO. 2 in which one to several amino acids have been deleted, substituted or added, the novel amino acid sequence having an ezrin-like domain, and DH and PH domains in the molecule.

Particularly, the above DNA includes DNA encoding a protein in which an amino acid sequence ranging from the 1st to 373rd amino acids in SEQ ID NO: 2 (ezrin-like domain) has homology of 85% or more to the amino acid sequence of the SEQ ID NO: 2, in which an amino acid sequence ranging from the 544th to 737th amino acids in SEQ ID NO: 2 (DH domain) has homology of 85% or more to the amino acid sequence of the SEQ ID NO: 2, and in which an amino acid sequence ranging from the 764th to 854th amino acids in SEQ ID NO: 2 (PH domain) has homology of 85% or more to the amino acid sequence of the SEQ ID NO: 2.

The DNA "encoding or coding for a protein" refers to DNA, if double-stranded, in which one of the complementary two strands has a nucleotide sequence coding for the protein. The DNA of the invention further includes DNA comprising a nucleotide sequence ranging from the 49th to 3183rd bases in the nucleotide sequence set forth in SEQ ID NO: 1, or a nucleotide sequence complementary thereto.

The invention also includes a DNA sequence encoding the CDEP protein, a DNA sequence including a strand complementary to the above sequence, or these sequences, a DNA sequence hybridized under standard conditions with the sequence or a fragment derived therefrom, and a DNA sequence which is not hybridized under standard conditions with the DNA sequence because of degeneracy of the genetic code, but which encodes a polypeptide having exactly the same amino acid sequence.

The "standard conditions" for hybridization refer to conditions generally used by people skilled in the art to detect specific hybridization signals, and those conditions mentioned, for example, in Sambrook et al, Molecular Cloning, Cold Spring Harbour Laboratory Press, New York, USA, Second Edition, 1989, preferably, so-called stringent hybridization-non-stringent washing conditions known among people skilled in the art and stated by Sambrook et al., more preferably, so-called stringent hybridization-stringent washing conditions. The stringent conditions preferably used in the invention are conditions under which a so-called specific hybrid is formed and a non-specific hybrid is not formed. It is difficult to express these conditions clearly as numerical values. However, concrete conditions include a temperature in the range of from the Tm of a hybrid of nucleic acids with high homology, e.g., a hybrid of completely matching nucleic acids, to a temperature 20°C lower than the Tm, or conditions at which DNA's having homology of 80% or more are hybridized, but nucleic acids having lower homology are not hybridized.

Furthermore, it is included in the invention to provide a DNA sequence which can be prepared by polymerase chain reaction (PCR) with the use of primers, designed based on the DNA sequence of SEQ ID NO: 1 of the sequence listing disclosed in the present specification, in accordance with the method well known in the art.

The DNA according to the invention can be synthesized on the basis of one of the nucleotides sequences of the DNA that have been determined by the invention as shown in Examples to be offered later on. The DNA can also be obtained from chromosomal DNA by PCR or hybridization using oligonucleotide primers or a probe prepared based on the nucleotide sequence. Alternatively, the DNA can be obtained by performing RT-PCR using cartilage mRNA, or by screening a cDNA library of cartilage or the like with the use of a polynucleotide, which has a nucleotide sequence encoding all or part of CDEP protein, as a probe.

The CDEP protein of the invention can be produced by inserting the aforementioned DNA of the invention into a publicly known expression vector to construct a recombinant plasmid, introducing this recombinant plasmid into a cell to obtain a transformant, culturing the transformant in a suitable medium to form and accumulate the protein of the invention in the culture, and collecting the protein from the culture.

As the cell and expression vector, a host-vector system usually used in the expression of a foreign protein can be used. Examples are combinations of procaryotic cells, such as Escherichia coli, and suitable expression vectors, and combinations of eucaryotic cells, such as mammalian cells, and suitable expression vectors. The culture media and culture conditions are suitably selected in harmony with the cells used.

The protein of the invention may be expressed as a fusion protein combined with other protein. Moreover, the protein of the invention may be expressed with its entire length, or may be expressed partially as a partial peptide.

The culture refers to a culture medium, and cells in the culture medium. Collection of the protein of the invention from the culture can be performed by a publicly known protein purification method using the activity, etc. of the protein of the invention as indicators.

If desired, the protein of the invention can be isolated from a heterogenetic organism by using the aforementioned nucleotide sequence information (in consideration of the codons used), by colony hybridization or southern blot hybridization using a probe synthesized in accordance with an amino acid sequence deduced from the nucleotide sequence, or by polymerase chain reaction (PCR) using primers synthesized by the information.

### (Antibody)

An antibody which can bind to the protein of the invention (hereinafter referred to as the antibody of the invention) can be prepared by the customary method using the protein of the invention as an antigen. The antibody of the invention may be polyclonal or monoclonal.

The protein of the invention may be used, unchanged, as an antigen, but is preferably used as an antigen after being joined to a carrier protein, such as keyhole limpet hemocyanin, bovine serum albumin, or ovalbumin, and/or in combination with an adjuvant.

The polyclonal antibody can be obtained by immunizing an animal, such as mouse, rabbit, guinea pig or sheep, with intraperitoneal or intravenous injection of the antigen, and taking the serum from the immunized animal.

The monoclonal antibody can be obtained, for example, in the following manner: An animal, such as mouse, rabbit, guinea pig or sheep, is immunized with subcutaneous, intraperitoneal or intravenous injection of the antigen. Then, the spleen or lymph node is removed, and a cell is taken therefrom. The cell is fused with a myeloma cell derived from an animal, preferably, allogeneic to the immunized animal to establish a hybridoma. From the resulting hybridoma, a cell strain which continuously produces specific antibodies to the antigen is chosen by repeating screening and cloning. The so selected cell strain is cultured in a suitable medium, and monoclonal antibodies are produced in the medium. Alternatively, the cell strain is cultured in vivo, e.g. intraperitoneally in the mouse, to produce monoclonal antibodies in ascitic fluid or the like.

Methods for purification of the resulting polyclonal and monoclonal antibodies include, for example, salting out by ammonium sulfate, ion exchange chromatography using a DEAE cellulose column or the like, affinity chromatography using a protein A column or the like, and immunoadsorbent chromatography. The antibody of the invention can be detected, for example, by immunoassay using the protein of the invention and a labeled antibody.

The antibody of the invention may be a fragmented one as long as the antigen-binding site (Fab) is conserved. An example of the fragmented antibody is an Fab-containing fragment obtained by decomposing the antibody with a protease which does not decompose the antigen-binding site, such as papain.

The antibody of the invention may have been labeled by being bound to a labeling substance. The labeling substance is not restricted, if normally usable for labeling a protein. Its examples are enzymes, isotopes, and fluorescent materials.

A method according to the invention for screening a regulator of cell differentiation induction using the protein of the invention may, for example, comprise adding the substance in question to a cell having differentiation potency, such as tetracarcinoma, and investigating what actual changes occurred in the cell, for instance, whether the cell differentiated, based on the shape of the cell and/or the expression of a marker gene. Alternatively; the screening method of the invention may be to add various medicines, which have been chemically synthesized, to the cell, and search for agents which can change the amount of expression of CDEP.

### (Kit for detection of differentiated chondrocytes, Method for detection of differentiated chondrocytes)

The method of the invention for detection of differentiated chondrocytes uses the aforementioned gene of the invention which is specifically expressed in differentiated chondrocytes.

Concretely, (a) the CDEP protein of the invention is used in a publicly known method to enable differentiated chondrocytes to be specifically detected. Alternatively, (b) the DNA of the invention, or its derivative is used in a publicly known method to enable differentiated chondrocytes to be specifically detected. Further alternatively, (c) the antibody of the invention is used in a publicly known method to enable differentiated chondrocytes to be specifically detected. Any combination of these methods can be used as the method of the invention.

### (Method for screening a regulator of cell differentiation induction, Kit for screening a regulator of cell differentiation induction)

The method of the invention for screening a regulator of cell differentiation induction uses the aforementioned gene of the invention which is specifically expressed in a differentiated chondrocytes.

Concretely, (a) the CDEP protein of the invention is used in a publicly known method to enable a regulator of cell differentiation induction to be screened. Alternatively, (b) the DNA of the invention is used in a publicly known method to enable a regulator of cell differentiation induction to be screened. Further alternatively, (C) the CDEP protein of the invention is used in a publicly known method to enable a regulator of cell differentiation induction to be screened. Any combination of these methods can be used as the method of the invention.

More concretely, the method for screening an anti-tumor agent as a regulator of cell differentiation induction may, for example, be to add various chemically synthesized agents to a cell cancerized by introduction of the aforesaid CDEP cDNA deficient on the N-terminal side, and search which of the agents return the cell to normal.

The invention further includes a regulator of cell differentiation induction which can be obtained by the above screening method.

### (Biological functions of CDEP)

The biological functions of CDEP obtained by the invention are summarized here. CDEP has an ezrin-like domain, and DH and PH domains in one molecule. In this view, CDEP may work by the following mechanism: The Ezrin-like domain dissociates Rho GDI from Rho, and then the DH and PH domains perform GDP-GTP exchange reaction in one molecule to regulate the activity of Rho, thereby controlling cell shape. Hence, CDEP is considered to be a very advantageous factor for activation of Rho among Rho GEFs.

### Examples

The present invention will now be described with reference to Examples to be offered below. PCR primers used in the Examples were synthesized by an ordinary automatic method of synthesis.
For type II collagen: CATACCGGTAAGTGGGGCAAGACTG, TGCCCAGTTCAGGTCTCTTA
For aggrecan: TGCTACTTCATCGACCCCAT, AAAGACCTCACCCTCCATCT
For CMP: GTCGATTACGTGGAGAGCTA, ATGAACTTCTTCACCAGCTC
For GAPDH: GTCAAGGCTGAGAACGGGAA, TCCACCACCCTGTTGCTGTA
For DEC1: ATCAGACCCAGCTCCCAAAG, CACAGACCCAGCTCCCAAAC
For CDEP: CCTTCAGGAAAACTCGTGTC, TTGGAGTTGTGTGTGGTCAG
For CDEP cDNA fragment: GCCAAAATAGTCACCTTCCACGAGG, CCTTCAGGAAAACTCGTGTC, AAACGRAAGAAYGTRTGRTGYTCWACACA, TTCCAGCTCCTAGAGATTGC, TCGTCTTCGCTCTCCTCAAT, CGGGTAACAAGCAGGCGGACGGA

### (Example 1)

Culture of differentiated chondrocytes was performed in the following manner in accordance with the method described in Biochemical and Biophysical Research Communications 236, 294-298 (1997), M. Shen, T. Kawamoto, W. Yan, K. Nakamasu, M. Tamagami, Y. Koyano, M. Noshiro, and Y. Kato:

From a human fetus spontaneously aborted at about 25 weeks of pregnancy (obtained from Norman Bethune University of Medical Sciences), an epiphysical cartilage on the femur side of the knee joint was obtained. From this cartilage, chondrocytes were isolated by the same method as described in Simomura et al. (1975), Calcif. Tissue Res. 19, 179-187, except that cartilage slices were incubated for 3 hours in an α-modified Eagle's medium (α-MEM) containing 3 mg/ml of collagenase (type IA, Sigma). The obtained cells were seeded on type I collagen coated dishes at a density of 1x10⁵ cells/dish. The cells were cultured in α-MEM (10 ml/dish) containing 10% bovine fetal serum, 50 µg/ml ascorbic acid, 32 units/ml of penicillin, and 40 µg/ml of streptomycin. When the cells became subconfluent, dibutyryl cAMP (dbcAMP) (1 mM) was added to the culture medium. The cells were cultured for 2 weeks or more in the presence and absence of dbcAMP, and then morphological observation of the cells was made. Also, the cells were harvested, fixed with ethanol, and stained with toluidine blue.

The chondrocytes cultured in the presence of dbcAMP took a spherical form, and were rich in extracellular matrix. Whereas the chondrocytes cultured in the absence of dbcAMP were spindle-shaped, similar to fibroblasts, and deficient in extracellular matrix.

Staining with toluidine blue, which selectively stains sulfated proteoglycan, satisfactorily stained the chondrocytes cultured in the presence of dbcAMP, but scarcely stained the chondrocytes cultured in the absence of dbcAMP.

The expression of mRNA of type II collagen, aggrecan and CMP as molecule markers was examined by RT-PCR. The expression of the molecule marker in the presence of dbcAMP was compared with that in the absence of dbcAMP. It was suggested tat a differentiated state was maintained in the presence of dbcAMP (Fig. 6).

Thus, the chondrocytes cultured in the presence of dbcAMP were admitted to maintain a differentiated state as cartilage, namely, a differentiated phenotype.

The above effect of dbcAMP was examined for dose dependency. This effect was found to increase dose dependently, and peaked at 0.3 to 0.5 mM.

Chondrocytes were cultured in the same manner as described above, except that bFGF (0.4 ng/ml) and TGF-β (3 ng/ml) were used instead of dbcAMP. bFGF and TGF-β are reported to stabilize or stimulate the expression of the differentiated phenotype of rabbit and chicken chondrocytes. The expression of the differentiated phenotype of the human-derived chondrocytes was not maintained with the use of bFGF and TGF-β.

### (Example 2) Gene specifically expressed in chondrocytes in a differentiated state

From the chondrocytes cultured in the presence of dbcAMP and in the absence of dbcAMP as described in Example 1, total RNA was extracted by the guanidine thiocyanate/cesium trifluoroacetate method. Poly(A)⁺RNA was concentrated using Oligotex-dT30 (Roche). Clones encoding mRNA, which is expressed in differentiated chondrocytes (+dbcAMP), but not in dedifferentiated chondrocytes (-dbcAMP), were selected by subtractive hybridization. That is, cDNA derived from mRNA of the differentiated chondrocytes was hybridized with an excess amount of cDNA derived from mRNA of the dedifferentiated chondrocytes, by use of PCR Select cDNA Subtraction Kit (Clontech). cDNA which is not hybridized, namely, which is expressed in a differentiated state, was amplified by suppression PCR. The resulting PCR product was cloned into a T tail vector pGEM-T (Promega), and nucleotide sequencing of about 120 clones was performed. For further analysis, one clone was selected, and the corresponding protein product was named CDEP.

Fig. 2A and Fig. 2B show the results of northern blotting analysis of CDEP mRNA expression in various human fetal tissues by the use of a 1.6 kb fragment of CDEP as the probe. Here, the transcript of 5.0 kb was also detected by northern blotting analysis. This finding suggests that in addition to the PCR product derived from 3.5 kb mRNA, a larger band induced from 5.0 kb mRNA probably hybridizable with the CDEP probe was found in RACE-PCR as well. However, the nucleotide sequencing of the product corresponding to the transcript of 5.0 It has not been performed yet.

The expression pattern of CDEP mRNA in cultured chondrocytes and various human tissues by northern blotting analysis showed that two main bands, 3.5 kb and 5.0 kb, were detected in cyclic AMP dependent differentiated chondrocytes, as illustrated in Fig. 2A, but they were not detected in fibroblasts without cyclic AMP. In fetal tissues, the highest level of CDEP mRNA was observed in the brain and the spleen, and low to intermediate levels of CDEP mRNA were noted in the heart, liver and intestine (Fig. 2A). In adult tissues, the amount of expression was intermediate in the kidney, testis, and lung, and considerably small in the heart, liver and intestine (Fig. 2B).

Northern blotting was performed in the following manner: The total RNA (10 µg) was electrophoresed on a 1% agarose gel containing formaldehyde, and transferred onto Hybond-N Membrane (Amersham).

Fetal human chondrocytes cultured in the presence and absence of Bt2cAMP, various fetal human tissues (Fig. 2A), and adult tissues (Fig. 2B) were electrophoresed on a 1% agarose gel containing formaldehyde, and transferred onto a nylon membrane. The membrane was hybridized with a CDEP probe (CDEP) or a glyceraldehyde-3-phosphate dehydrogenase probe (GAPDH). To examine the tissue distribution, total RNA of various fetal human tissues were supplied by Dr. Li Yu of Norman Bethune University of Medical Sciences. A 1.6 kb fragment of CDEP was labeled with [³²P]dCTP, and used as a hybridization probe. The membrane was washed with 0.2 × SSC containing 0.5% SDS for 30 minutes at 65°C. A biomax X-ray film was exposed to light through the washed membrane using a sensitizing film at -70°C.

Nucleotide sequencing of the full length of CDEP cDNA was performed in the following manner: CDEP full length cDNA was isolated by the rapid amplification cDNA ends (RACE) method using Marathon cDNA Amplification Kit (Clontech). In detail, double-stranded cDNA was joined to Marathon cDNA adaptor, and subjected to suppression PCR. The reaction was carried out using an adaptor primer, and a gene specific primer designed for CDEP on the basis of the nucleotide sequence of CDEP. The amplified cDNA sample was separated using a 4% polyacrylamide gel, and DNA of major bands was extracted from the gel and subloned into pGEM-T. The subcloned plasmid double-stranded DNA and a series of synthetic oligonucleotides were used as a sequencing template and specific primers, respectively. DNA sequencing was performed by the Sanger method using Sequenase 7-deaza-dGTP DNA Sequencing Kit (Amersham), or ABI PRISM 310 Autosequencer (Perkin Elmer).

The so determined nucleotide sequence of CDEP cDNA and an amino acid sequence deduced therefrom are set forth in SEQ ID NO: 1. Only the amino acid sequence is set forth in SEQ ID NO: 2. CDEP cDNA has an open reading frame of 3135 bp. The length of 3287 bp, 3306 bp or 3442 bp, excluding the poly(A) region, agrees highly with the size (3.5 kb) of the mRNA obtained by the aforementioned northern blotting analysis. Since an in-frame stop codon exists in the 5'-region (nucleotide 34), the first ATG is admitted as an initiation codon.

### (Example 3) Expression of CDEP mRNA in rabbit costal cartilage growth plate slices

Total RNA was extracted from each of layers, i.e., costal cartilage growth plate slices (a portion near the bone side was divided into three parts designated as G3, G2 and G1) and a resting cartilage layer (called R) of a 4-weeks-old male Japanese albino rabbit. The extract was examined for the expression level of CDEP mRNA by the RT-PCR method. The expression levels of type X collagen, alkaline phosphatase, and proteoglycan aggrecan, differentiation markers of chondrocytes, in the respective layers were confirmed by RT-PCR.

Extraction of total RNA was performed from the cartilage tissue by guanidine-cesium chloride ultracentrifugation. The RT-PCR method used SUPERSCRIPT Preamplification System (GIBCO BRL). For DNA amplification, DNA Thermal Cycler (Perkin-Elmer Cetus) was used.

Reverse transcription was performed using the total RNA (0.5 µg) extracted from the cartilage tissue, and amplification was carried out using an upstream specific primer (5'-TCACTTCGTGGTTTCAGAGC-3') and a downstream specific primer (TCGTCTTCGCTCTCCTCAAT-3') that were designed based on the nucleotide sequence of CDEP to obtain a CDEP cDNA fragment. The conditions for the amplification reaction were set to carry out a denaturation reaction (95°C, 1 min), annealing and extension (65°C, 3 min), and 20 cycles of amplification were performed. The resulting DNA was electrophoresed for 15 minutes at 100 V on a 1% agarose gel.

PCR southern blotting was performed in the following manner: The electrophoresed DNA in the gel was denatured in a 0.5N NaOH solution containing 1.5M NaCl, and then transferred onto a nylon membrane Nytran0.45 (Schleicher & Schuell) with the use of 6 × SSC. This membrane was subjected to a hybridization reaction for 16 hours at 68°C in a hybridization solution containing a [³²P]labeled CDEP probe labeled with Oligolabelling Kit (Pharmacia Biochem). After the reaction, the membrane was washed, and autoradiography was performed. The radioactivity was measured with BAS2000 Image Analyzer (Fujix).

Rabbit costal cartilage growth plate chondrocytes were cultured in the following manner: The growth plates were separated from the costal cartilages of the 4-weeks-old male Japanese albino rabbit. They were digested with 0.1% EDTA, 0.1% trypsin containing PBS and 0.05% collagenase containing DMEM, and chondrocytes were isolated. Six-well tissue culture plates were seeded with 300,000 cells per well, and the cells were cultured with alpha MEM containing 10% bovine fetus serum. The culture medium was replaced at intervals of 2 days, starting on the 5th day of culture.

The expression of CDEP mRNA in the rabbit costal cartilage growth plate chondrocytes culture system was investigated by extracting total RNA from the cell layer on the 6th, 10th, 14th, 18th, 22nd and 26th days of culture, and determining the expression level of CDEP mRNA by RT-PCR.

The induction of CDEP mRNA by PTH in the rabbit costal cartilage growth plate chondrocytes culture system was investigated by adding PTH (1-84) (1x10⁻⁷M) after 6, 10, 14, 18, 22 and 26 days of culture, incubating the system for a further 24 hours, then extracting total RNA, and determining the expression level of CDEP mRNA at each stage by RT-PCR.

The time course of the induction of CDEP mRNA expression by PTH and cAMP was investigated by adding PTH (1-84) (1x10⁻⁷M) and Bt₂cAMP in rabbit costal cartilage growth plate chondrocytes on the 12th day of culturing, extracting total RNA 1, 3, 6, 12 and 24 hours later, and determining the expression level of CDEP mRNA at each time point by RT-PCR.

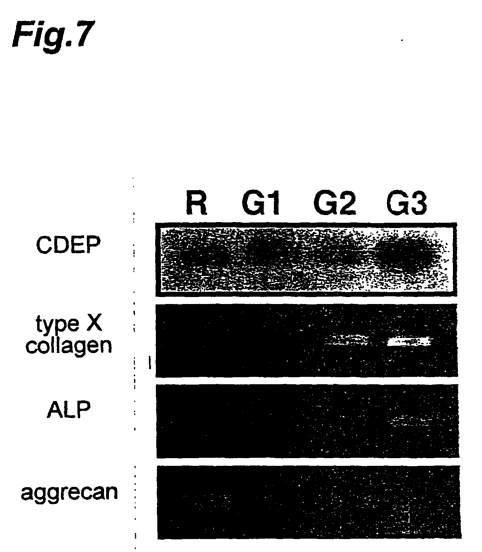

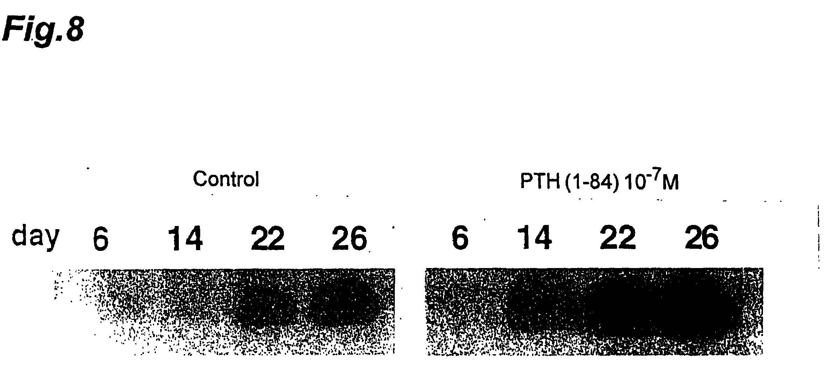

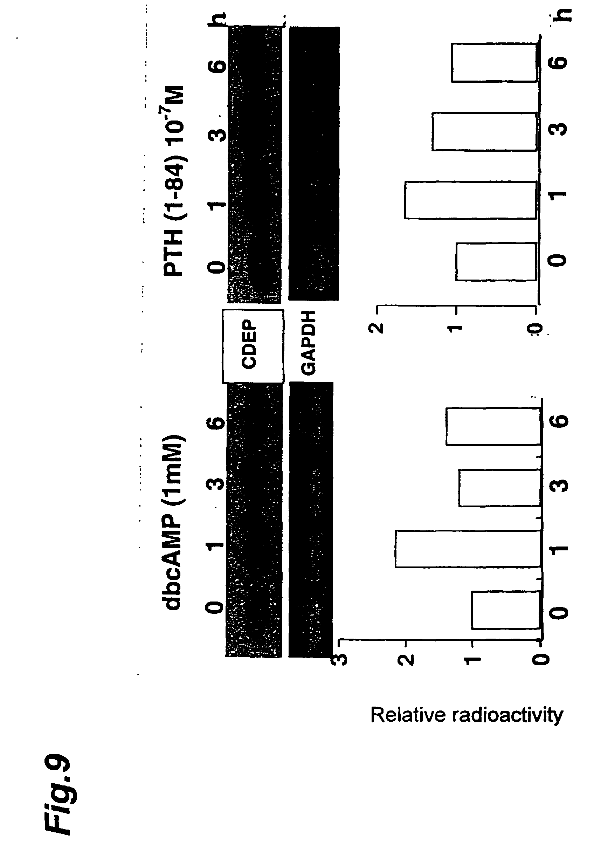

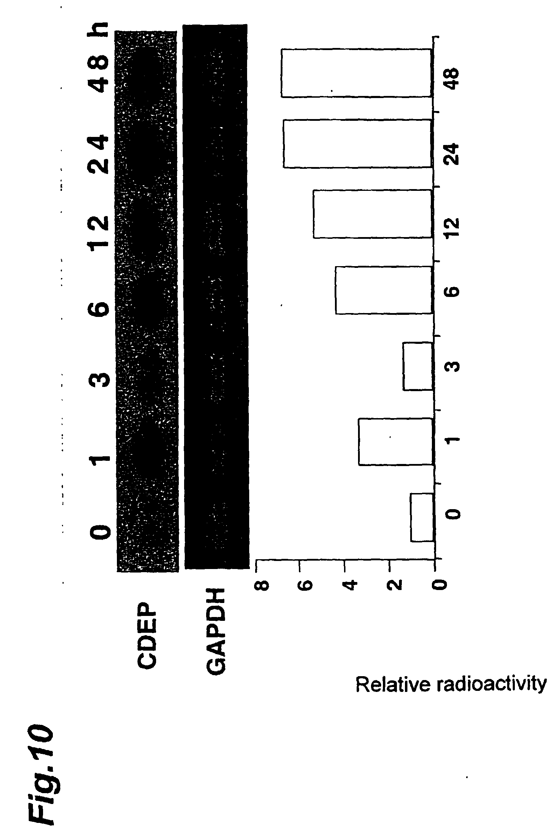

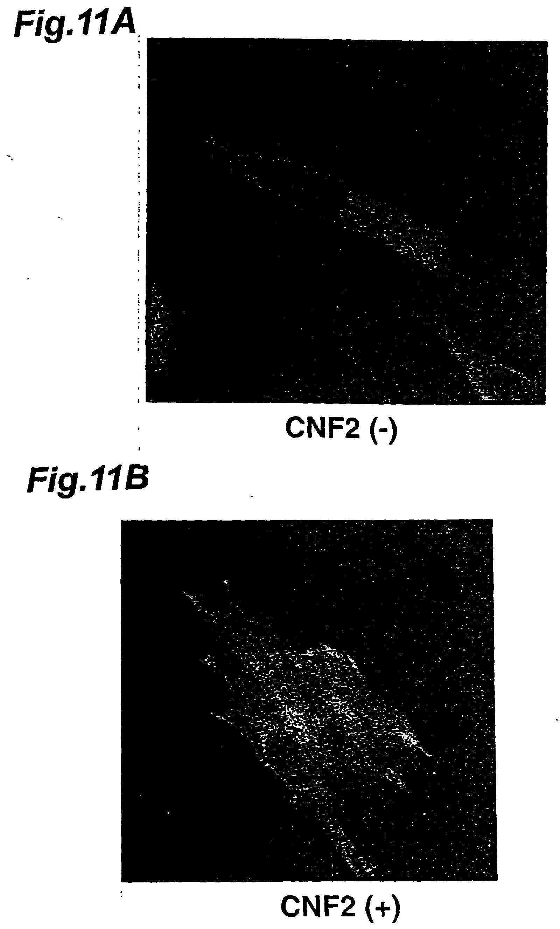

The results of the foregoing experiments and the results given below (Example 4) gave the following findings: (1) In the in vivo cartilage tissue, more CDEP is expressed in the hypertrophic layer showing the expression of type X collagen and ALP, the markers of terminal differentiation of chondrocytes (hypertrophy, calcification) (Fig. 7, Fig. 11A, Fig. 11B). (2) In the in vitro chondrocytes culture system, more CDEP mRNA is expressed in the hypertrophic stage and later (on the 22nd day of culturing and later), and the amount of expression is further increased upon the addition of PTH using cAMP as a second messenger (Fig. 8). Furthermore, (3) the level of CDEP mRNA rises transiently at a very early stage (as early as 1 hour later) in short-term induction during the period of from 0 to 6 hours after addition of cAMP and PTH, and then rises over time (Fig. 9). Moreover, the expression in the period of from 0 to 48 hours after addition of PTH becomes biphasic (Fig. 10).

### (Example 4) Intracellular localization of CDEP by immunofluorescence staining

Immunofluorescence staining was performed to investigate the localization of CDEP in the cell with the use of MRC5, a human lung fibroblast cell line. That is, 3,000 cells were seeded on a 1x1 cm cover glass, and cultured for 24 hours in DMEM. Then, cytotoxic necrotizing factor 2 (CNF2) derived from enteropathogenic E. coli was added, and the so treated cells were cultured for a further 12 hours together with a CNF2-free control group. The cultures were washed with PBS, and then fixed with 2% paraformaldehyde for 30 minutes at room temperature. Then, the system was washed with PBS again, treated for 3 minutes with 0.5% Triton X-100, and further washed with PBS. Then, the system was incubated for 1 hour in PBS containing 5% sheep serum at room temperature to block nonspecific binding. After washing with PBS containing 0.05% Tween 20 (PBST), the system was incubated for 2 hours at room temperature along with anti-CDEP antiserum diluted 1:100 with PBS. Then, after washing with PBST, the system was incubated for 30 minutes at room temperature under light-shielded conditions together with FITC-labeled anti-rabbit sheep IgG (Cappel) diluted 1:1000. After washing with PBST, the system was observed with a confocal laser microscope (LSM410, Carl Zeiss) (Figs. 11A, 11B).

In the absence of CNF2, the cytoplasm was stained as a whole. In the presence of CNF2, polymerization of actin took place. CDEP was localized in filamentous processes and ruffling sites of the cell membrane that occurred on this occasion.

### Industrial Applicability

As described above, the present invention provides genes expressed specifically in differentiated human-derived chondrocytes. These genes are important not only in analyzing the mechanism of differentiation or degeneration of a cartilage, but also in developing gene therapies for osteoarthritis and rheumatoid arthritis. Furthermore, CDEP according to the invention is expressed in cartilage and other tissues, and thus its important actions in such other tissues are suggested.

That is, CDEP of the invention, in view of its structural features, serves as a regulatory factor for a cytoskeleton binding to protein on a cell membrane, and also has a structure characteristic of an epistatic factor for regulating the functions of Rho. By controlling an actin-series cytoskeleton, i.e., activating Rho in response to signals from upstream to control signal transduction downstream, CDEP may maintain cell shape or regulate cell adhesion, cell aggregation, or cell motility. From the aspect of being a regulatory factor for cytoskeleton, CDEP may play important roles in the reconstruction of a cytoskeleton which occurs as a result of stimulation by cell growth factors, or cell transformation. For chondrocytes, changes in cell shape are closely related to differentiation, and it is known that differentiated chondrocytes takes a spherical form, while dedifferentiated chondrocytes is spindle-shaped. Hence, if the shape of a cell can be changed to a spherical form by accelerating or suppressing the functions of CDEP, it may be possible to induce or maintain the differentiation of chondrocytes. Gene introduction with the expectation of such an action may make it possible to control the differentiated state of chondrocytes with deviating functions (dedifferentiated chondrocytes) in arthropathies, including osteoarthritis.

Among other Rho GEF family members, some are known to work as oncogenes by arousing such abnormalities as the lack of an amino acid on the N-terminal side. They are reported to enhance the infiltrating ability of cancer. If it is found that active type mutation, such as deficiency in part of a CDEP gene, causes canceration of a cell, a novel therapeutic drug with a specific suppressive effect may be developed with CDEP as a target.

Also, it is conceivable to prepare a dominant negative mutant of CDEP, and introduce its gene into a cancer cell to inhibit the activity of the cancer cell, thereby achieving cancer treatment. In other words, CDEP is likely to be useful in elucidating the mechanism of control of the functions of the entire Rho family.

Ezrin binds to CD44, etc. on a cell membrane at its N-terminus, and binds to actin on its C-terminal side, thereby taking part in cytoskeletal regulation. Rho is considered to regulate this binding. CDEP, which has an ezrin-like domain at the N-terminal, and DH and PH domains at the C-terminal, may bind to protein on the cell membrane at the N-terminal, and activate Rho on the C-terminal side. Thus, CDEP may be a regulatory factor for a cytoskeleton which binds to a cell membrane, and also be a factor for regulating signals to Rho by feedback of the state of the cytoskeleton.

## Claims

1. A protein specifically expressed in differentiated chondrocytes, comprising an ezrin-like domain, a Db1 domain, and a pleckstrin domain.

2. A protein having an amino acid sequence set forth in SEQ ID NO: 2 in the sequence listing.

3. A protein comprising an amino acid sequence set forth in SEQ ID NO: 2 in the sequence listing in which one to several amino acids have been deleted, substituted or added, the protein being specifically expressed in differentiated chondrocytes, and the protein being such that
(1) the amino acid sequence of a portion of the protein corresponding to an amino acid sequence ranging from the 1st to 374th amino acids in SEQ ID NO: 2 in the sequence listing has homology of 85% or more to the amino acid sequence ranging from the 1st to 374th amino acids in the SEQ ID NO: 2,
(2) the amino acid sequence of a portion of the protein corresponding to an amino acid sequence ranging from the 544th to 737th amino acids in SEQ ID NO: 2 in the sequence listing has homology of 85% or more to the amino acid sequence ranging from the 544th to 737th amino acids in the SEQ ID NO: 2, and
(3) the amino acid sequence of a portion of the protein corresponding to an amino acid sequence ranging from the 764th to 854th amino acids in SEQ ID NO: 2 in the sequence listing has homology of 85% or more to the amino acid sequence ranging from the 764th to 854th amino acids in the SEQ ID NO: 2.

4. DNA encoding the protein according to anyone of claims 1 to 3.

5. A gene comprising DNA shown in the following (a) or (b):
(a) DNA comprising a nucleotide sequence ranging from the 49th to 3,183rd bases in a nucleotide sequence set forth in SEQ ID NO: 1 in the sequence listing; and
(b) DNA which is hybridized under stringent conditions with DNA having a nucleotide sequence ranging from the 49th to 3,183rd bases in a nucleotide sequence set forth in SEQ ID NO: 1 in the sequence listing, and which encodes a protein specifically expressed in differentiated chondrocytes.

6. DNA having a part of or all of a nucleotide sequence set forth in SEQ ID NO: 1 in the sequence listing, or having a nucleotide sequence complementary to the part of or all of the nucleotide sequence.

7. An antibody against the protein according to any one of claims 1 to 3.

8. A kit for screening a regulator of cell differentiation induction, the kit comprising at least one of the following (a) to (c) as an active ingredient:
(a) the protein according to any one of claims 1 to 3;
(b) the DNA according to any one of claims 4 to 6; and
(c) the antibody according to claim 7.

9. A methods for screening a regulator of cell differentiation induction, the method comprising using the kit according to claim 8.

10. The method for screening claimed in claim 9, wherein the regulator of cell differentiation induction is an anti-tumor agent.
